Europäisches Patentamt

European Patent Office

Office européen des brevets

(11) Veröffentlichungsnummer: **0 303 115**
**A2**

(12) **EUROPÄISCHE PATENTANMELDUNG**

(21) Anmeldenummer: **88112282.4**

(22) Anmeldetag: **29.07.88**

(51) Int. Cl.⁴: **A61L 2/18**

(30) Priorität: **14.08.87 DE 3727169**

(43) Veröffentlichungstag der Anmeldung:
**15.02.89 Patentblatt 89/07**

(84) Benannte Vertragsstaaten:
**BE DE FR IT**

(71) Anmelder: **Wolf, Hans-Joachim**
**Theodor-Schweitzer-Strasse 1**
**D-7137 Sternenfels 1(DE)**

(72) Erfinder: **Wolf, Hans-Joachim**
**Theodor-Schweitzer-Strasse 1**
**D-7137 Sternenfels 1(DE)**

(74) Vertreter: **Frank, Gerhard, Dipl.-Phys.**
**Patentanwälte Dr. F. Mayer & G. Frank**
**Westliche 24**
**D-7530 Pforzheim(DE)**

(54) **Desinfektionsgerät für medizinische Geräte, insbesondere Endoskope.**

(57) Ein Desinfektionsgerät für medizinische Geräte, insbesondere Endoskope, weist einen flachen Behälter zur Aufnahme der Sterilisierflüssigkeit auf, in den ein mit dem Endoskop versehener Trägereinsatz vertikal zur Desinfektion einschiebbar ist. Der Trägereinsatz besteht hierbei vorzugsweise aus einem Drahtgitter mit zum Teil kreisbogenförmigen Haltebügeln, durch die die einzelnen Teile des Endoskops gegeneinander fixiert werden, wobei jedoch eine weitgehende Freiheit beim Einlegen der Schlauchteile insofern bestehen bleibt, als daß auch Endoskope mit verschieden langen Schlauchteilen ohne Schwierigkeiten untergebracht werden können. Durch die vertikale Anordnung kann durch eine entsprechende Wahl der Füllhöhe des Behälters mit Desinfektionsflüssigkeit auch einfach erreicht werden, daß die Endoskope entweder vollständig oder unter Aussparung des Beobachtungs- und/oder Versorgungsteils in die Desinfektionsflüssigkeit eintauchen.

Die vertikale Anordnung benötigt außerdem nur ein Minimum an Platz, so daß dieses Desinfektionsgerät beispielsweise für die Wandmontage geeignet ist.

Es ist auch möglich, das Desinfektionsgerät als integrale Einheit aufzubauen, wobei der Deckel des Behälters gleichzeitig als Gehäuse für Versorgungsaggregate und Versorgungsanschlüsse für das Endoskop ausgebildet ist.

## Desinfektionsgerät für medizinische Geräte, insbesondere Endoskope.

Die Erfindung betrifft ein Desinfektionsgerät für medizinische Geräte mit Untersuchungsschläuchen, insbesondere für Endoskope, mit einem Behälter zur Aufnahme der Sterilisierflüssigkeit und einem Trägereinsatz mit Auflagevorrichtungen für das zu desinfizierende Endoskop, das in den Behälter einbringbar ist.

Ein derartiges Desinfektionsgerät ist aus der DE-OS 34 05 268 bekannt.

In der Praxis wurden im Lauf der letzten Jahre verschiedene Typen von Endoskopen eingesetzt; bei den älteren Modellen darf nur der zur eigentlichen medizinischen Untersuchung verwendete Untersuchungsschlauch der Einwirkung der Desinfektionsflüssigkeit ausgesetzt werden; die anderen Teile des Endoskops (insbesondere Bedienteil mit Okular und Versorgungsschlauch) waren von der Desinfektion ausgeschlossen.

Beim vorbekannten Desinfektionsgerät besteht der Behälter aus einer Wanne, in die der Trägereinsatz einsetzbar ist; in den Trägereinsatz ist das zu desinfizierende Endoskop in seiner Gesamtheit einlegbar, d.h., daß sämtliche Endoskop-Teile beim Desinfiziervorgang von der Desinfektionsflüssigkeit umspült sind.

Zur Desinfektion der oben erwähnten älteren Endoskop-Modelle muß der Trägereinsatz entsprechend geformt sein, um die Auflage der nicht desinfizierbaren Endoskopteile außerhalb der Desinfektionsflüssigkeit zu gewährleisten, beispielsweise muß der Versorgungsschlauch in eine Halterung im Deckel des Sterilisiergeräts eingehängt werden. Die Handhabung des vorbekannten Geräts bei der Desinfektion dieses älteren Endoskops läßt daher einige Wünsche offen.

Die handelsüblichen Endoskope weisen Versorgungsschläuche und Untersuchungsschläuche mit verschiedenen Längen auf, so daß bei einer allzu starren Festlegung des Schlauchverlaufes im Trägereinsatz des vorbekannten DesinfektionsgerätesSchwierigkeiten bei dem Einlegen bestimmter Endoskoptypen auftreten können.

Letztlich benötigt das vorbekannte Gerät einen erheblichen Platzbedarf, da die Wanne in der Regel in einem separaten Gestell oder Desinfektionswagen untergebracht werden muß.

Infolge der im wesentlichen waagerechten Lagerung des Endoskops und der dadurch gebildeten großen Oberfläche der Desinfektionsflüssigkeit, verdunstet auch die Desinfektionsflüssigkeit relativ schnell, was bei der Handhabung des vorbekannten Gerätes ohne den Deckel zu einer nicht wünschenswerten Inhalierung dieser Dämpfe durch die Bedienungsperson führen kann.

Es sind auch Desinfektionseinrichtungen bekannt, die im wesentlichen aus vertikal angeordneten Röhren bestehen, in die der Untersuchungsschlauch des Endoskops einhängbar ist.

Hierbei ist die Verdunstung der Desinfektionsflüssigkeit zwar minimiert, jedoch können in diesen engen Röhren nur die Untersuchungsschläuche desinfiziert werden.

Aufgabe der Erfindung ist es daher, das vorbekannte Desinfektionsgerät so weiter zu entwickeln, daß eine platzsparende und universelle Desinfektion aller handelsüblichen Endoskope ermöglicht wird, d.h., daß wahlweise das Bedienteil und der Versorgungsschlauch des Endoskops mit desinfiziert werden können oder auch nicht und daß sämtliche Längen der Versorgungs- und Untersuchungsschläuche aufgenommen werden können. Erfindungsgemäß wird diese Aufgabe dadurch gelöst, daß das Endoskop im Trägereinsatz hängend gehalten ist, der vertikal in den Behälter einschiebbar ist.

Durch die Verwendung eines Trägereinsatzes eröffnet sich die Möglichkeit, durch geeignete Auflagebereiche die Unterbringung verschiedenster Endoskoptypen zu ermöglichen, durch die vertikale Anordnung dieses Trägereinsatzes in einem Behälter kann dieser sehr schmal ausgebildet werden und beispielsweise an einer Wand fest montiert werden.

Durch die schmale Ausbildung des Behälters, dessen Querschnitt nur geringfügig über dem minimalen Querschnitt des eingehängten Endoskops bemessen sein muß, wird auch eine geringere Oberfläche der Desinfektionsflüssigkeit geschaffen und deren Verdunstung eingeschränkt.

In weiterer Ausgestaltung ist vorgesehen, daß der Trägereinsatz ein Drahtgitter mit mehreren definierten Auflagebereichen für das Endoskop ist, die im unteren Teil des Drahtgitters als kreisbogenförmige Haltebügel ausgebildet sind. Zur Sicherung des Endoskops auf diesen Auflagebereichen können Arretierriegel oder Gummistränge vorgesehen sein, die abschnittsweise über das Endoskop geklappt oder gespannt werden.

Die ausschließliche Verwendung einer Drahtgitter-Konstruktion sowohl zur Schaffung einer "Unterlage", als auch zur Schaffung der Auflagebereiche hat den Vorteil, daß die von der Desinfektionsflüssigkeit benetzte Oberfläche der Drahtgitterstäbe sehr gering gehalten werden kann, wodurch der unvermeidliche Verlust von Desinfektionsflüssigkeit durch Benetzung von nicht zum Endoskop gehörigen Oberflächen gering gehalten werden kann.

Eine vorteilhafte Ausgestaltung sieht weiterhin vor, daß oberhalb des Trägereinsatzes und fest verbunden mit diesem Versorgungsaggregate zur Desinfektion angeordnet sind, deren gemeinsames Gehäuse die Bedienungselemente aufnimmt und als Deckel für den Behälter dient. Durch die feste Zuordnung der Versorgungsaggregate zum Trägereinsatz können die Versorgungsleitungen zur Durchspülung des Endoskops kurz gehalten werden, da sie jeweils am gemeinsamen Gehäuse angeschlossen werden können.

Die Integration von Behälter, Trägereinsatz und Versorgungsaggregate erlaubt außerdem die Schaffung eines weitgehend autarken Standgerätes zur Desinfektion von Endoskopen, das auch verfahrbar ausgestaltet sein kann, insbesondere wird es auch dadurch möglich, dem Behälter ein Aggregat zur Erzeugung einer Strömung innerhalb der Desinfektionsflüssigkeit zuzuordnen, wenn dies erforderlich ist; dies kann entweder durch Einbeziehung eines solchen Aggregates in den Behälter selbst oder durch eine entsprechende Anschlußmöglichkeit erreicht werden.

Ferner schafft die feste Zuordnung von Trägereinsatz und Deckel mit integrierten Versorgungsaggregaten die Möglichkeit, mit dem eingehängten Endoskop nach der Desinfektion im Behälter weitere Behälter, beispielsweise ein sogenanntes Neutralisationsbecken, anzufahren, ohne daß hierzu umständliches Hantieren mit den Versorgungsleitungen zur Durchspülung des Endoskops erforderlich wäre, da diese, ohnehin nur kurz, jedem "Ortswechsel" des Endoskops mitmachen, bis das vollständig "behandelte" Endoskop zum Gebrauch entnommen wird.

Weitere Ausgestaltungen sind Unteransprüchen zu entnehmen.

Zwei Ausführungsbeispiele des erfindungsgemäßen Desinfektionsgerätes werden nun anhand von Zeichnungen näher erläutert, es zeigen:

Figur 1: eine Aufsicht auf das erste Ausführungsbeispiel des Desinfektionsgerätes,

Figur 2: eine Seitenansicht des Desinfektionsgerätes gemäß Figur 1,

Figur 3: eine Vorderansicht des zweiten Ausführungsbeispiels des Desinfektionsgerätes, und

Figur 4: eine Seitenansicht des zweiten Ausführungsbeispiels gemäß Figur 3.

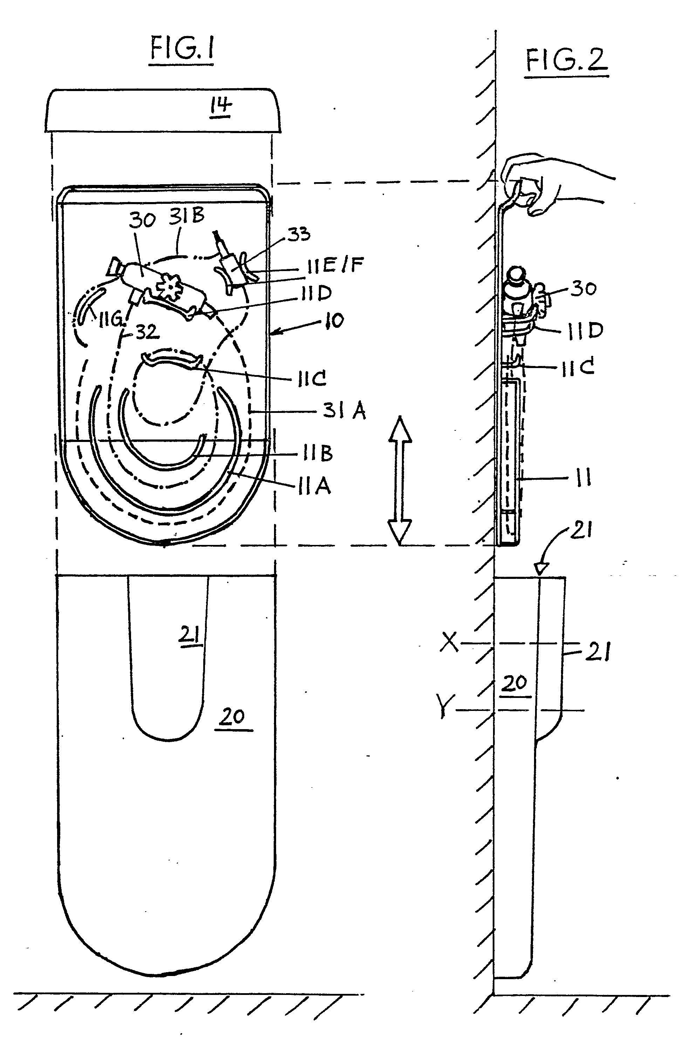

Bei dem in den Figuren 1 und 2 dargestellten Ausführungsbeispiel handelt es sich um ein Desinfektionsgerät zur Wandmontage. Es besteht in seinen wesentlichen Teilen aus einem flachen Behälter 20, der fest an der Wand installiert ist, einem Trägereinsatz 10 zur Aufnahme eines Endoskops 30 mit dessen Schläuchen 31A,31B und 32 mit Versorgungsteil 33 und schließlich einen Deckel 14 zur Abdeckung des Behälters 20, wenn sich der Trägereinsatz 10 im Behälter 20 befindet. Der Behälter 20 dient in bekannter Weise zur Aufnahme der Desinfektionsflüssigkeit; wie durch den Doppelpfeil angedeutet, wird der Trägereinsatz 10 mit dem zu desinfizierenden Endoskop in den Behälter 20 eingetaucht und nach der Desinfektion wieder entnommen.

Da das Beobachtungsteil 30 des Endoskops in der Regel infolge der Einstell- und Bedienungsorgane eine gewisse Dicke nicht unterschreitet, ist am Behälter 20 eine obere zentrale Ausbauchung 21 vorgesehen, die die vollständige Aufnahme des Beobachtungsteils 30 ermöglicht, ohne daß die benötigte Menge an Desinfektionsflüssigkeit, die durch das Volumen des Behälters 20 bestimmt ist, übermäßig vergrößert wird.

Die Berücksichtigung der Desinfektionsvorschriften für Endoskope, die vollständig in die Desinfektionsflüssigkeit eintauchen dürfen und solchen, bei denen dies nicht möglich ist, kann bei dem dargestellten Gerät einfach durch eine entsprechende Füllhöhe X oder Y der Desinfektionsflüssigkeit im Behälter berücksichtigt werden; es ist jedoch auch möglich, an (im Ausführungsbeispiel der Figuren 1 und 2 nicht dargestellten) vertikalen Führungsschienen für den Trägereinsatz 10 entsprechende Arretierungen vorzusehen, mit denen der Trägereinsatz 10 mehr oder weniger weit in den Behälter eintaucht, so daß nur der Untersuchungsschlauch von der Desinfektionsflüssigkeit zu liegen kommt. Der Versorgungsschlauch wird dann oberhalb des Flüssigkeitsspiegels in entsprechende Haltebügel des Trägereinsatzes 10 eingehängt.

Der Trägereinsatz 10 besteht aus einem Drahtgitter, von dem in der Figur 1 lediglich die zur Ortsfixierung der Schläuche des Endoskops vorgesehenen Haltebügel 11A...11G dargestellt sind. Insbesondere an den oberen Haltebügeln 11C,11D sind diese in ihrem äußeren Bereich nach oben abgebogen, so daß sich eine sichere Auflage insbesondere für das Beobachtungsteil 30 und das Versorgungsteil 33 des Endoskops ergibt. Beim dargestellten Ausführungsbeispiel ist der gestrichelt dargestellte Beobachtungsschlauch 31A relativ kurz und ist deshalb unterhalb des unteren, kreisbogenförmigen Haltebügels 11A angeordnet, wogegen der längere, strichpunktiert dargestellte erste Versorgungsschlauch 32 sehr viel länger ist, dieser ist zwischen dem Haltebügel 11A,11B angeordnet und nochmals um den Haltebügel 11C gelegt. Bei Endoskopen mit abweichenden Schlauchlängen kann diese Anordnung ohne weiteres vertauscht werden, wobei also zweckmäßigerweise der jeweils längere Schlauch in den inneren Bereich des Trägereinsatzes angeordnet wird und der kürzere Schlauch in den äußeren Bereich.

Durch diese Anordnungsmöglichkeiten einerseits und die nur teilweise Führungsvorgabe für die Schläuche durch die Haltebügel 11 andererseits können somit alle Endoskoptypen mit unterschiedlicher Schlauchlänge angeordnet werden. Für solche Endoskoptypen mit zusätzlichem Versorgungsschlauch 31B ist ein weiterer Haltebügel 11G vorgesehen. Das Versorgungsteil 33 sitzt in zwei gegenüberliegenden Haltebügeln 11E,11F.

Dadurch, daß die Haltebügel zur Aufnahme des Beobachtungsteils 30 und des Versorgungsteils 33 etwa auf gleicher Höhe liegen und oberhalb der Haltebügel zur Fixierung der Schläuche angeordnet sind, kann die oben erwähnte wahlweise Desinfektion nur des Untersuchungsschlauchs oder auch aller Teile des Endoskops durch entsprechende Füllstandshöhe X,Y im Behälter 20 erzielt werden.

Das obere Ende des Trägereinsatzes 10 ist bügelförmig nach außen geneigt, so daß es als Handgriff zur leichten Handhabung des Trägereinsatzes 10 dient.

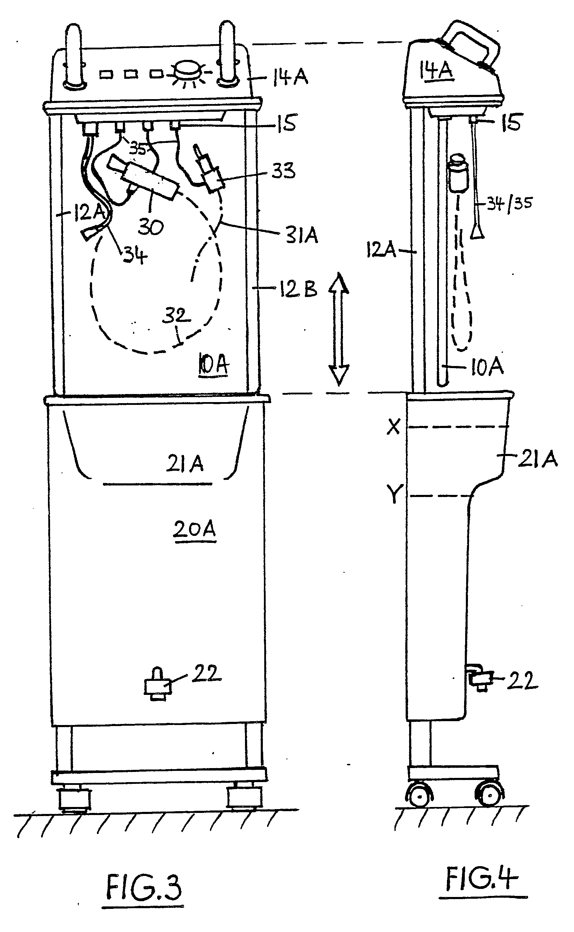

Bei dem in den Figuren 3 und 4 dargestellten zweiten Ausführungsbeispiel des erfindungsgemäßen Sterilisiergerätes handelt es sich um eine Kompaktbaueinheit, deren Aufbauprinzipien im wesentlichen dem ersten Ausführungsbeispiel der Figuren 1 und 2 entsprechen, so daß der Aufbau und das Zusammenwirken des Trägereinsatzes 10A und des Behälters 20A sowie die Halterung der Endoskopteile im einzelnen nicht mehr erläutert werden braucht.

Im Unterschied zum ersten Ausführungsbeispiel ist der Trägereinsatz 10A oben am Deckel 14A befestigt, der Deckel ist hierbei gleichzeitig als Gehäuse für Bedienungselemente und Versorgungsaggregate wie z.B. einer Pumpe ausgebildet und weist Versorgungsanschlüsse 15 auf, die mit den entsprechenden Anschlüssen der Endoskopteile durch kurze und stationäre Schlauchteile 35 verbindbar sind. Über einen Ansaugschlauch 34 (oder ein Ansaugrohr) wird die Desinfektionsflüssigkeit mittels der eingebauten Pumpe über die drei dargestellten Schlauchteile 35 durch das Innere der Endoskopteile gepumpt. Das Deckelteil 14A ist über vertikale seitliche Führungsschienen 12A,12B mitsamt Trägereinsatz 10A in den Behälter 20A einfahrbar. Dadurch wird automatisch ein vollständiger Abschluß der Desinfektionsflüssigkeit erreicht. Durch diese Konzeption wird eine weitgehend autarke Sterilisiereinheit geschaffen, die lediglich noch die üblichen Strom- und Wasseranschlüsse benötigt und mit einem Minimum an Platz auskommt.

Am unteren Ende des Behälters 20A ist ein Ablaufstutzen 22 vorgesehen, mit dem nach einer gewissen Anzahl von Desinfektionsvorgängen die Desinfektionsflüssigkeit abgelassen werden kann. In diesem unteren Bereich kann bei Bedarf zur Verbesserung der Umspülung der zu reinigenden Endoskopteile auch eine Umwälzvorrichtung angeordnet sein, die beispielsweise mittels einer Flügelschraube der Desinfektionsflüssigkeit eine beispielsweise zirkulierende Strömung aufprägt.

## Ansprüche

1. Desinfektionsgerät für medizinische Geräte mit Untersuchungsschläuchen, insbesondere für Endoskope, mit einem Behälter zur Aufnahme der Sterilisierflüssigkeit und einem Trägereinsatz mit Auflagevorrichtungen für das zu desinfizierende Endoskop, das in den Behälter einbringbar ist, dadurch gekennzeichnet, daß das Endoskop (30...33) im Trägereinsatz (10) hängend gehalten ist, der vertikal in den Behälter (20) einschiebbar ist.

2. Desinfektionsgerät nach Anspruch 1, dadurch gekennzeichnet, daß der Trägereinsatz (20) ein Drahtgitter mit mehreren definierten Auflagebereichen für das Endoskop (30...33) ist.

3. Desinfektionsgerät nach Anspruch 2, dadurch gekennzeichnet, daß die Auflagebereiche im unteren Teil des Drahtgitters (10) als kreisbogenförmige Haltebügel (11A,11B) ausgebildet sind.

4. Desinfektionsgerät nach Anspruch 1, dadurch gekennzeichnet, daß der Behälter (20) den Trägereinsatz (10) mit dem eingelegten Endoskop (30...33) unter Beibehaltung einer oberen Öffnung (21) eng umschließt.

5. Desinfektionsgerät nach Anspruch 1, dadurch gekennzeichnet, daß in den Innenraum des Behälters (20A) vertikale Führungsschienen für den Trägereinsatz (10A) führen.

6. Desinfektionsgerät nach Anspruch 5, dadurch gekennzeichnet, daß am Trägereinsatz (10) seitliche Führungsschienen angebracht sind.

7. Desinfektionsgerät nach Anspruch 1 bis 6, dadurch gekennzeichnet, daß der Behälter (20A) als verfahrbarer Standbehälter ausgebildet ist.

8. Desinfektionsgerät nach Anspruch 1 bis 7, dadurch gekennzeichnet, daß oberhalb des Trägereinsatzes (10) Versorgungsaggregate zur Desinfektion angeordnet sind, deren gemeinsames Gehäuse die Bedienungselemente aufnimmt und als Deckel (14A) für den Behälter (20A) ausgebildet ist, wenn der Trägereinsatz (10A) mit dem Endoskop (30...33) in den Behälter (20A) eingeschoben ist.

9. Desinfektionsgerät nach Anspruch 1, dadurch gekennzeichnet, daß dem Behälter (20,20A) ein Aggregat zur Erzeugung einer Strömung innerhalb der Desinfektionsflüssigkeit zugeordnet ist.

# FIG.1

# FIG.2

FIG.3

FIG.4